# EUROPÄISCHE PATENTSCHRIFT

(11) **EP 0 684 048 B1**
(45) Veröffentlichungstag und Bekanntmachung des Hinweises auf die Patenterteilung: **02.09.1998**
(21) Anmeldenummer: 95105696.9
(22) Anmeldetag: 15.04.1995
(51) Int. Cl.: A61M 16/00

(54) **Vorrichtung zur Sauerstofftherapie**
Oxygen therapy device
Dispositif d'oxygénothérapie

(30) Priorität: 18.04.1994 DE 9406407 U
(43) Veröffentlichungstag der Anmeldung: 29.11.1995
(73) Patentinhaber: Schneider, Peter, 56759 Laubach (DE)
(72) Erfinder: Schneider, Peter, 56759 Laubach (DE)
(74) Vertreter: Rehmann, Klaus-Thorsten, Dipl.-Ing.

(56) Entgegenhaltungen:
- EP-A- 0 125 424
- EP-A- 0 183 593
- WO-A-87/06040
- FR-A- 988 242
- FR-A- 2 251 340
- US-A- 3 400 712
- US-A- 4 517 982

## Beschreibung

Die Erfindung betrifft eine Vorrichtung zur Sauerstofftherapie eines Patienten, dem im wesentlichen reiner Sauerstoff in die Atemwege eingeleitet wird, mit einem über eine Steuervorrichtung regelbaren, nur während der Einatmungsphase des jeweiligen Atemzyklus des Patienten geöffneten Durchflußregler, wobei die Steuerung mit einer den Atmungsverlauf des Patienten ermittelnden Meßeinrichtung in Verbindung steht.

Eine Sauerstofftherapie dient zur Unterstützung der geschädigten Atmung, z. B. bei Frischoperierten, Herz- und Lungenkranken und Kranken mit Atemnot. Die Sauerstofftherapie erfolgt herkömmlicherweise mit Hilfe einer Vorrichtung, die im wesentlichen aus einem Sauerstoffbehälter und einem daran befestigten Schlauch besteht, der zum Patienten geführt wird. Zur Regulierung des dem jeweiligen Patienten zuströmenden Sauerstoffes ist ferner ein Durchflußregler vorgesehen, welcher üblicherweise derart eingestellt wird, daß dem Patienten ständig z.B. 6 Liter Sauerstoff pro Minute zufließen. Damit nicht unnötigerweise Sauerstoff zugeführt wird, wenn dieser vom Patienten nicht aufgenommen werden kann, ist die Steuervorrichtung mit einer den Atemverlauf ermittelnden Meßeinrichtung verbunden. Eine solche Vorrichtung ist in der EP-A- 0 125 424 offenbart. Zur Ermittlung der Atemfrequenz wird ein Brustgurt mit einem Sensor verwendet, der das Anheben bzw. Senken des Brustkorbes beim Ein- und Ausatmen ermittelt. Auch die US-A-3,400,712 offenbart eine Vorrichtung zur Sauerstofftherapie eines Patienten, bei der zur Ermittlung des Atemzyklus ein Brustgurt Verwendung findet. Solche Sensoren arbeiten beispielsweise mit Dehnungsmeßstreifen.

Ein Brustgurt ist dann unvorteilhaft, wenn der Patient flach atmet oder die sogenannte Bauchatmung betreibt, und sich der Brustkorb nicht ausreichend hebt und senkt. Wenn sich der Patient bewegt, besteht die Gefahr, daß der Gurt verrutscht und der Atemzyklus nicht mehr ermittelt werden kann. Aus Sicherheitsgründen muß die Vorrichtung so eingestellt sein, daß für den Fall, daß über längere Zeit keine Atmung ermittelt wird, der Durchflußregler permanent geöffnet wird. Der mit einem Brustgurt versehene Patient muß folglich recht ruhig liegen, um die Funktionsfähigkeit nicht zu beeinträchtigen.

Die genannten Nachteile haben dazu geführt, daß die beschriebenen Vorrichtungen nie in die Praxis umgesetzt worden sind. Für die Sauerstofftherapie in Krankenhäusern wird heute ausschließlich permanent Sauerstoff zugeführt. Das heißt, während des gesamten Atemzyklus (Einatmungsphase, Ausatmungsphase sowie eventueller kurzer Pause) wird Sauerstoff zugeführt. Es wird deshalb etwa 2/3 mehr Sauerstoff zugeführt, als der Patient tatsächlich nutzen kann.

Von dieser Problemstellung ausgehend soll die Meßvorrichtung zur Ermittlung des Atemzyklus verbessert werden.

Zur Problemlösung dient als Meßeinrichtung eine Impedanzmeßeinrichtung, die die Änderungen des elektrischen Wechselstromwiderstandes zwischen mindestens zwei auf dem Brustkorb des Patienten angeordneten Elektroden während des Atmungsverlaufs mißt.

Eine Impedanzmeßeinrichtung an sich ist bereits bei Überwachungsgeräten bekannt, die in der Intensivmedizin Verwendung finden. Mit diesen Geräten, die vielfach gleichzeitig auch mit einer EKG-Meßeinrichtung gekoppelt sind, soll dem Pflegepersonal auf der Intensivstation signalisiert werden, wenn der Patient aufhört zu atmen. Da die Impedanzmeßeinrichtung sehr sensibel reagiert, wird beispielsweise in der US-A-4,517,982 vorgeschlagen, eine solche Einrichtung zur Überwachung der Herz-Lungenfunktion insbesondere bei Neugeborenen zu verwenden.

Überraschenderweise hat sich gezeigt, daß die an sich nur für das Monitoring, also an eine Anzeige- und/oder Signaleinrichtung gelieferten Signale auch als Steuersignale verwendet werden können.

Mit der Erfindung wird es nun möglich, die bestehenden Vorrichtungen in Krankenhäusern einfach umzurüsten. Auch das Meßinstrument für die Impedanzmessung steht in aller Regel zur Verfügung. Es muß folglich nur ein Durchflußregler und ein entsprechendes Steuergerät in die bestehenden Leitungen integriert werden. Durch die Erfindung wird der nun seit langem in der Praxis bestehende Bedarf endlich zur Zufriedenheit gelöst.

Vorteilhaft ist es, wenn die Signale einer Elektrokardiogramm-Meßeinrichtung zur Gewinnung der Stellsignale des Durchflußreglers herangezogen werden. Die gemessenen elektrischen Ströme der Herz- und der Atmungsmuskulatur können mittels eines eines Frequenzfilters getrennt werden, wobei die Ströme der Atmungsmuskulatur zur Gewinnung der Stellsignale herangezogen werden.

Eine weitere Verringerung der Sauerstoffzufuhr des Patienten kann dadurch erreicht werden, daß die Steuervorrichtung den Durchflußregler immer dann schließt, wenn dem jeweiligen Patienten eine vorgebbare Sauerstoffmenge zugefüht wurde (z.B. 200 ml), wobei vorteilhafterweise die zugeführte Sauerstoffmenge zu Beginn der Einatmungsphase des jeweiligen Atmungszyklus am größten ist und dann absinken kann. Denn nur die während etwa der ersten Hälfte der Einatmungsphase eingeatmete Luft nimmt am Gasaustausch in der Lunge teil. Die danach nachströmende Atemluft dient in erster Linie lediglich dem Auffüllen der größeren oberen Luftwege, wie Rachen, Luftröhre und Bronchien, und braucht somit nicht zusätzlich mit Sauerstoff angereichert zu werden, da sie bei der Ausatmung als erstes unverbraucht wieder ausgeatmet wird.

Weitere Einzelheiten und Vorteile der Erfindung werden im folgenden anhand eines in Figuren dargestellten Ausführungsbeispieles erläutert. Es zeigen:
- Figur 1 -: ein Blockschaltbild der erfindungsgemäßen Vorrichtung,
- Figur 2 -: den Verlauf des mit einer Impedanzmeßeinrichtung gemessenen Körperwiderstandes im Bereich des Brustkorbes beim Atmen eines Patienten und
- Figur 3 bis 5 -: verschiedene aus dem in Figur 2 dargestellten Widerstandsverlauf abgeleitete Stellsignale für die AnSteuerung des Durchflußreglers.

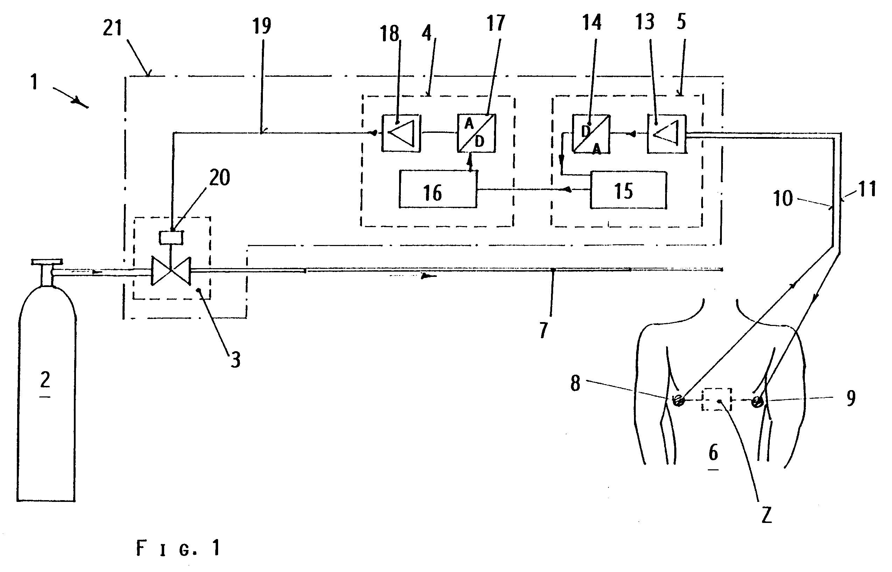

In Fig.1 ist mit 1 eine erfindungsgemäße Vorrichtung bezeichnet, die im wesentlichen aus einer Sauerstofflasche 2, einem elektromechanischen Durchflußregler 3, einer den Durchflußregler ansteuernden Steuervorrichtung 4 und einer an sich bekannten digitalen Impedanzmeßeinrichtung 5, wie sie in der Impedanzpneumographie verwendet wird, besteht (aus Übersichtlichkeitsgründen ist die für die Impedanzmessung erforderliche Wechselspannungsquelle nicht dargestellt).

Mit dem Bezugszeichen 6 ist ein nur schematisch angedeuteter Patient bezeichnet. Der Patient 6 ist über eine Schlauchleitung 7 mit dem Durchflußregler 3 verbunden. Außerdem sind auf seinem Brustkorb zwei Elektroden 8, 9 befestigt, die mittels elektrischer Leitungen 10, 11 mit der Impedanzmeßeinrichtung 5 verbunden sind.

Fig.2 zeigt den Verlauf der Änderung der Körperimpedanz Δ Z zwischen den beiden Elektroden 8 und 9 (Fig.1) beim Atmen. Dabei erhöht sich die Impedanz während des Einatmungsvorganges durch Verdrängen des Blutes. Mit 12 ist in Fig.2 ein Atemzyklus und mit 120 die Einatmungsphase dieses Atemzyklusses gekennzeichnet.

In der Meßeinrichtung 5 (Fig.1) werden die entsprechenden Meßwerte in einem Verstärker 13 verstärkt, dann in einem Analog-Digital-Wandler 14 digitalisiert und schließlich mit Hilfe eines Prozessors 15 ausgewertet. Die digitalisierten Meßwerte werden dann der Steuervorrichtung 4 zugeführt, wo sie beispielsweise in einem weiteren Prozessor 16 bewertet und anschließend gegebenenfalls nach Umwandlung in einem Digital-Analog-Wandler 17 und Verstärkung in einem Verstärker 18 über eine Leitung 19 dem Steuereingang 20 des Durchflußeglers 3 zugeführt werden.

Den Verlauf entsprechender an dem Steuereingang 20 des Durchflußreglers 3 anliegender Stellwerte (Stellsignale) zeigen die Fig.3-5. Dabei zeigt Fig.3 den Verlauf eines Stellwertes, bei dem jeweils während der gesamten Einatmungsphase 120 der Durchflußregler 3 vollständig geöffnet ist und der Patient 6 entsprechend mit Sauerstoff versorgt wird.

Im Falle der Fig.4 bewirkt eine entsprechende Vorgabe, daß der Prozessor 16 bereits dann den Durchflußregler 3 abschaltet, wenn eine vorgegebene Sauerstoffmenge dem Patienten zugeführt wurde. Die Breite der einzelnen Stellsignale wird im Durchschnitt gegenüber den in Fig.3 dargestellten Signalen daher schmaler.

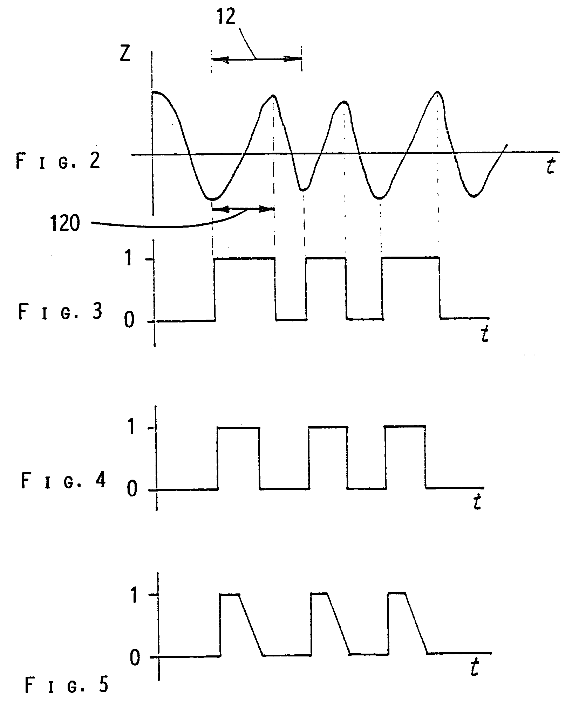

Fig.5 schließlich zeigt den Fall, daß das Stellsignal beim Beginn der Einatmung einen etwa konstanten Verlauf aufweist und daß es anschließend linear abfällt. Entsprechend diesem Verlauf ist auch die Sauerstoffversorgung des Patienten während der Einatmungsphase 120.

Die Erfindung ist selbstverständlich nicht auf das dargestellte Ausführungsbeispiel beschränkt. So kann es sich bei dem Durchflußregler 3 auch um ein elektromagnetisches oder ein hydraulisch gesteuertes System handeln. Der Aufbau der Steuervorrichtung 4 kann, je nach vorgegebener Betriebsart, wesentlich von dem in Fig.1 dargestellten Aufbau abweichen. Um etwa die in Fig.3 dargestellten Stellwerte zu erhalten, wird es häufig ausreichen, einen Schaltkreis zu verwenden, der jeweils bei ansteigender Flanke des Widerstandsverlaufes (Einatmungsphase) ein Setzsignal und bei abfallender Flanke ein Rücksetzsignal erzeugt. Auch eine Schwellenbewertung des in Fig.2 dargestellten Signales kann (z.B. softwaremäßig) erfolgen. Ferner braucht es sich bei der Steuervorrichtung und der Meßeinrichtung nicht notwendigerweise um getrennte Baueinheiten zu handeln. Vielmehr können beide Baueinheiten zu einer einzigen Baueinheit zusammengefaßt werden, was in Fig.1 durch die strichpunktierte Linie 21 angedeutet ist.

Schließlich ist die erfindungsgemäße Vorrichtung nicht nur zur Sauerstofftherapie von menschlichen Patienten geeignet, sondern kann mit geringen Abwandlungen auch bei einer Sauerstoffbehandlung von Tieren eingesetzt werden.

### Bezugszeichenliste

- 1: Vorrichtung
- 2: Sauerstofflasche
- 3: Durchflußregler
- 4: Steuervorrichtung
- 5: Meßeinrichtung, Impedanzmeßeinrichtung
- 6: Patient
- 7: Sauerstoffschlauch
- 8,9: Elektroden
- 10,11: elektrische Leitungen
- 12: Atemzyklus
- 13: Verstärker
- 14: Analog-Digital-Wandler
- 15: Prozessor
- 16: Prozessor
- 17: Digital-Analog-Wandler
- 18: Verstärker
- 19: Leitung
- 20: Steuereingang
- 21: Baueinheit
- 120: Einatmungsphase
- Z: Körperimpedanz
- t: Zeit

## Patentansprüche

1. Vorrichtung zur Sauerstofftherapie eines Patienten (6), dem im wesentlichen reiner Sauerstoff in die Atemwege eingeleitet wird, mit einem über eine Steuervorrichtung (4) regelbaren, nur während der Einatmungsphase des jeweiligen Atemzyklus des Patienten (6) geöffneten Durchflußregler (3), wobei die Steuervorrichtung (4) mit einer den Atmungsverlauf des Patienten (6) ermittelnden Meßeinrichtung (5) in Verbindung steht, **dadurch gekennzeichnet, daß** als Meßeinrichtung (5) ein Impedanzmeßeinrichtung dient, die die Änderungen des elektrischen Wechselstromwiderstandes (Δ Z) zwischen mindestens zwei auf dem Brustkorb des Patienten (6) angeordneten Elektroden (8,9) während des Atmungsverlaufes mißt.

2. Vorrichtung nach Anspruch 1, **dadurch gekennzeichnet, daß** als Meßeinrichtung (5) ein Gerät zur Aufnahme eines Elektrokardiogrammes (EKG-Meßeinrichtung) dient, bei dem die gemessenen elektrischen Ströme der Herz- und der Atmungsmuskulatur mittels eines Frequenzfilters trennbar sind, wobei die Ströme der Atmungsmuskulatur zur Gewinnung von Stellsignalen herangezogen werden.

3. Vorrichtung nach Anspruch 1 oder 2, **dadurch gekennzeichnet, daß** die Steuervorrichtung (4) Stellsignale erzeugt, die den Durchflußregler (3) immer dann schließen, wenn dem jeweiligen Patienten (6) eine vorgebbare Sauerstoffmenge zugeführt wurde.

4. Vorrichtung nach einem der Ansprüche 1 bis 3, **dadurch gekennzeichnet, daß** die Steuervorrichtung (4) den Durchflußregler (3) derart ansteuert, daß zu Beginn der Einatmungsphase (120) der Durchflußregler (3) maximal geöffnet ist, und daß er sich während der Einatmungsphase (120) dann allmählich schließt.

## Claims

1. Device for oxygen therapy of a patient (6) into whose respiratory tract substantially pure oxygen is introduced, with a flow regulator (3) that can be controlled via a control device (4) and that is open only during the inhalation phase of the particular respiratory cycle of the patient (6), the control device (4) being linked to a measuring device (5) that ascertains the course of respiration of the patient (6), characterised in that use is made, by way of measuring device (5), of an impedance-measuring device which measures, during the course of respiration, the changes in the electrical a.c. resistance (Δ Z) between at least two electrodes (8, 9) arranged on the thorax of the patient (6).

2. Device according to Claim 1, characterised in that use is made, by way of measuring device (5), of an instrument for recording an electrocardiogram (electrocardiographic measuring device), with which the measured electrical currents pertaining to the cardiac musculature and respiratory musculature can be separated by means of a frequency filter, the currents pertaining to the respiratory musculature being utilised for the purpose of obtaining actuating signals.

3. Device according to Claim 1 or 2, characterised in that the control device (4) generates actuating signals which close the flow regulator (3) whenever a specifiable quantity of oxygen has been supplied to the patient (6) in question.

4. Device according to one of Claims 1 to 3, characterised in that the control device (4) controls the flow regulator (3) in such a way that the flow regulator (3) is maximally open at the start of the inhalation phase (120) and then closes gradually during the inhalation phase (120).

## Revendications

1. Dispositif pour l'oxythérapie d'un patient (6) auquel on introduit de l'oxygène sensiblement pur dans les voies respiratoires, comprenant un régulateur d'écoulement (3) régulable par l'intermédiaire d'un dispositif de commande (4) et qui n'est ouvert que pendant la phase inspiratoire de chaque cycle respiratoire du patient (6), le dispositif de commande (4) étant en liaison avec un dispositif de mesure (5) relevant le processus respiratoire du patient (6), caractérisé en ce qu'un dispositif de mesure d'impédance sert de dispositif de mesure (5) et mesure les variations de la résistance au courant électrique alternatif (ΔZ) entre au moins deux électrodes (8, 9) disposées sur la cage thoracique du patient (6) pendant le processus respiratoire.

2. Dispositif selon la revendication 1, caractérisé en ce que comme dispositif de mesure (5) sert un appareil de relevé d'un électrocardiogramme (dispositif de mesure ECG), dans lequel les courants électriques mesurés de la musculature cardiaque et respiratoire peuvent être séparés au moyen d'un filtre de fréquence, les courants de la musculature respiratoire étant utilisés pour produire des signaux de réglage.

3. Dispositif selon la revendication 1 ou 2, caractérisé en ce que le dispositif de commande (4) produit des signaux de réglage, qui ferment le régulateur d'écoulement (3) chaque fois qu'une quantité d'oxygène que l'on peut fixer à l'avance a été envoyée au patient concerné (6).

4. Dispositif selon l'une des revendications 1 à 3, caractérisé en ce que le dispositif de commande (4) commande le régulateur d'écoulement (3) de manière que le régulateur d'écoulement (3) soit ouvert au maximum au début de la phase inspiratoire (120), puis se ferme progressivement ensuite pendant la phase inspiratoire (120).
